(19) Europäisches Patentamt
European Patent Office
Office européen des brevets

(11) Publication number: **0 652 220 A1**

(12) **EUROPEAN PATENT APPLICATION published in accordance with Art. 158(3) EPC**

(21) Application number: **93913536.4**

(51) Int. Cl.[6]: **C07D 501/59**, A61K 31/545

(22) Date of filing: **17.06.93**

(86) International application number:
**PCT/JP93/00816**

(87) International publication number:
**WO 94/02491 (03.02.94 94/04)**

(30) Priority: **22.07.92 JP 195074/92**

(43) Date of publication of application:
**10.05.95 Bulletin 95/19**

(84) Designated Contracting States:
**AT BE CH DE DK ES FR GB GR IE IT LI LU MC NL PT SE**

(71) Applicant: **TAISHO PHARMACEUTICAL CO. LTD**
**24-1 Takata 3-chome**
**Toshima-ku**
**Tokyo 171 (JP)**

(72) Inventor: **ONODERA, Akira Taisho Pharmaceutical Co., Ltd.**
**24-1, Takata 3-chome**
**Toshima-ku**
**Tokyo 171 (JP)**
Inventor: **MIYAZAWA, Tomoki Taisho Pharmaceutical Co., Ltd.**
**24-1, Takata 3-chome**
**Toshima-ku**
**Tokyo 171 (JP)**
Inventor: **MORIMOTO, Shigeo Taisho Pharmaceutical Co., Ltd.**
**24-1, Takata 3-chome**
**Toshima-ku**
**Tokyo 171 (JP)**
Inventor: **HATAYAMA, Katsuo Taisho Pharmaceutical Co., Ltd.**
**24-1, Takata 3-chome**
**Toshima-ku**
**Tokyo 171 (JP)**

(74) Representative: **Weisert, Annekäte, Dipl.-Ing. Dr.-Ing.**
**Patentanwälte**
**Kraus Weisert & Partner**
**Thomas-Wimmer-Ring 15**
**D-80539 München (DE)**

(54) **CEPHALOSPORIN DERIVATIVE.**

(57) A novel cephalosporin derivative represented by general formula (I), which has a potent antibacterial activity, a low toxicity and a high oral absorbability, and a nontoxic salt thereof, wherein $R^1$ and $R^3$ represent each hydrogen or lower alkyl, and $R^2$ represents hydrogen or a group hydrolyzable in vivo. A drug containing this compound as the active ingredient has not only a potent antibacterial activity against various pathogenic bacteria including gram positive and negative bacteria but also an excellent oral absorbability and a low toxicity, thus being useful as an antibacterial for oral administration.

EP 0 652 220 A1

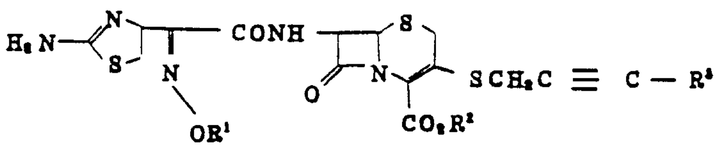 (I)

## INDUSTRIAL FIELD

The present invention relates to novel cephalosporin derivatives having a substituted thio group at the 3-position, and particularly to novel compounds of oral cephalosporin derivatives having potent antibacterial activity and the salts thereof.

## BACKGROUND ART

Antibiotics of cephalosporin series are very effective as the therapeutical agents of bacterially infectious diseases due to their superior antibacterial action and high safety. Especially, cephem derivatives having an aminothiazole group on the substituent of the amino group at the 7-position have been developed in recent years because of having potent antibacterial activity and wide antibacterial spectrum.

The present inventors disclose some cephem derivatives having good oral absorbability in Japanese Patent Kokai 1-308289.

## DISCLOSURE OF THE INVENTION

Many antibiotics of cephalosporin series useful as drugs are known, and many of them are administered parenterally because of low oral absorbability. Of the oral cephalosporin drugs used clinically, cephalexin and cefachlor have a phenylglycylamino group at the 7-position and show high oral absorbability, but have extremely inferior antibacterial activity against gram-negative bacteria to injectional cephalosporin drugs.

Oral cephalosporin drugs of which carboxylic acid at the 4-position is esterified in order to elevate the oral absorbability as so-called prodrug have been recently developed. These compounds have stronger antibacterial activity against gram-negative bacteria due to the introduction of an aminothiazolyl group at the 7-position than cephalexin, but remain the drawbacks of lowered antibacterial activity against gram-positive bacteria and the oral absorbability which is not so good.

As a result of extensive researches for solving the above-mentioned problem, the present inventors have successed to synthesize novel cephalosporin derivatives having both strong antibacterial activity and high oral absorbability, and have accomplished the present invention.

The present invention relates to cephalosporin derivatives represented by the formula:

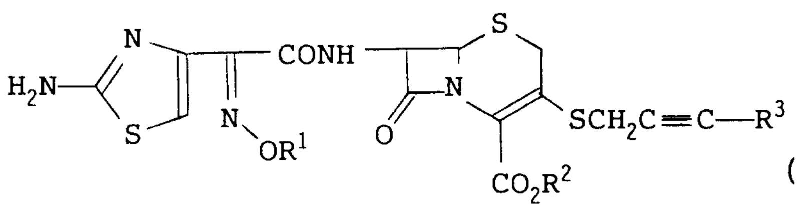

(I)

(wherein $R^1$ and $R^3$ are each a hydrogen atom or a lower alkyl group, and $R^2$ is a hydrogen atom or an in vivo hydrolyzable group) and non-toxic salts thereof.

In the present invention, the in vivo hydrolyzable group refers to one which is known in the prodrug techniques of the $\beta$-lactam drugs such as a 5-methyl-1,3-dioxacyclopent-4-en-2-on-4-ylmethyl group, an acetoxymethyl group, a pivaloyloxymethyl group, a 1-pivaloyloxyethyl group, a 1-acetoxyethyl group, a 3-phthalidyl group, a 1-(cyclohexyloxycarbonyloxy)ethyl group or a 1-(isopropoxycarbonyloxy)ethyl group. Examples of the lower alkyl group as defined for $R^1$ and $R^3$ are a methyl group, an ethyl group and a propyl group.

The non-toxic salt of the compound of Formula (I) of the present invention refers to pharmacologically acceptable salts such as, for example, salts with inorganic bases including sodium, potassium, magnesium or ammonium; salts with organic bases such as triethylamine or cyclohexylamine; salts with basic amino acids such as arginine or lysine; salts with mineral acids such as sulfuric acid, hydrochloric acid or phosphoric acid; or salts with organic acids such as acetic acid, lactic acid, tartaric acid, fumaric acid, maleic acid, trifluoroacetic acid, p-toluenesulfonic acid or methanesulfonic acid.

In Formula (I), $R^1$ is preferably a hydrogen atom, $R^2$ is preferably a hydrogen atom, a group known in the prodrug techniques of the $\beta$-lactam drugs such as a pivaloyloxymethyl group or a 1-(isopropoxycarbonyloxy)ethyl group, $R^3$ is preferably a hydrogen atom, and the non-toxic salt is preferably maleate.

The compounds of Formula (I) of the present invention are those in the forms of geometric isomers [E-form and Z-form] derived from the oxyimino group at the 7-position side chain, and both isomers are included within the scope of the present invention, but the Z-form is preferred.

The compound of Formula (I) of the present invention can be prepared, for example, by the following Preparation Method I or II.

Preparation Method I

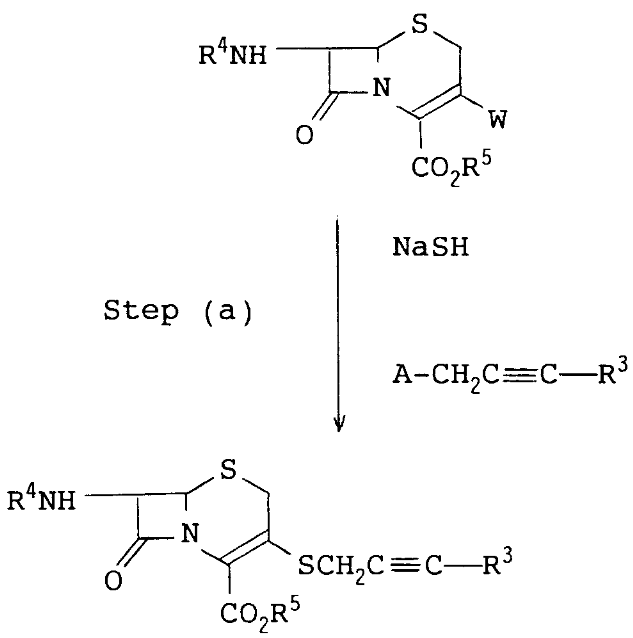


(II)

(III)

(IV)

(IV)

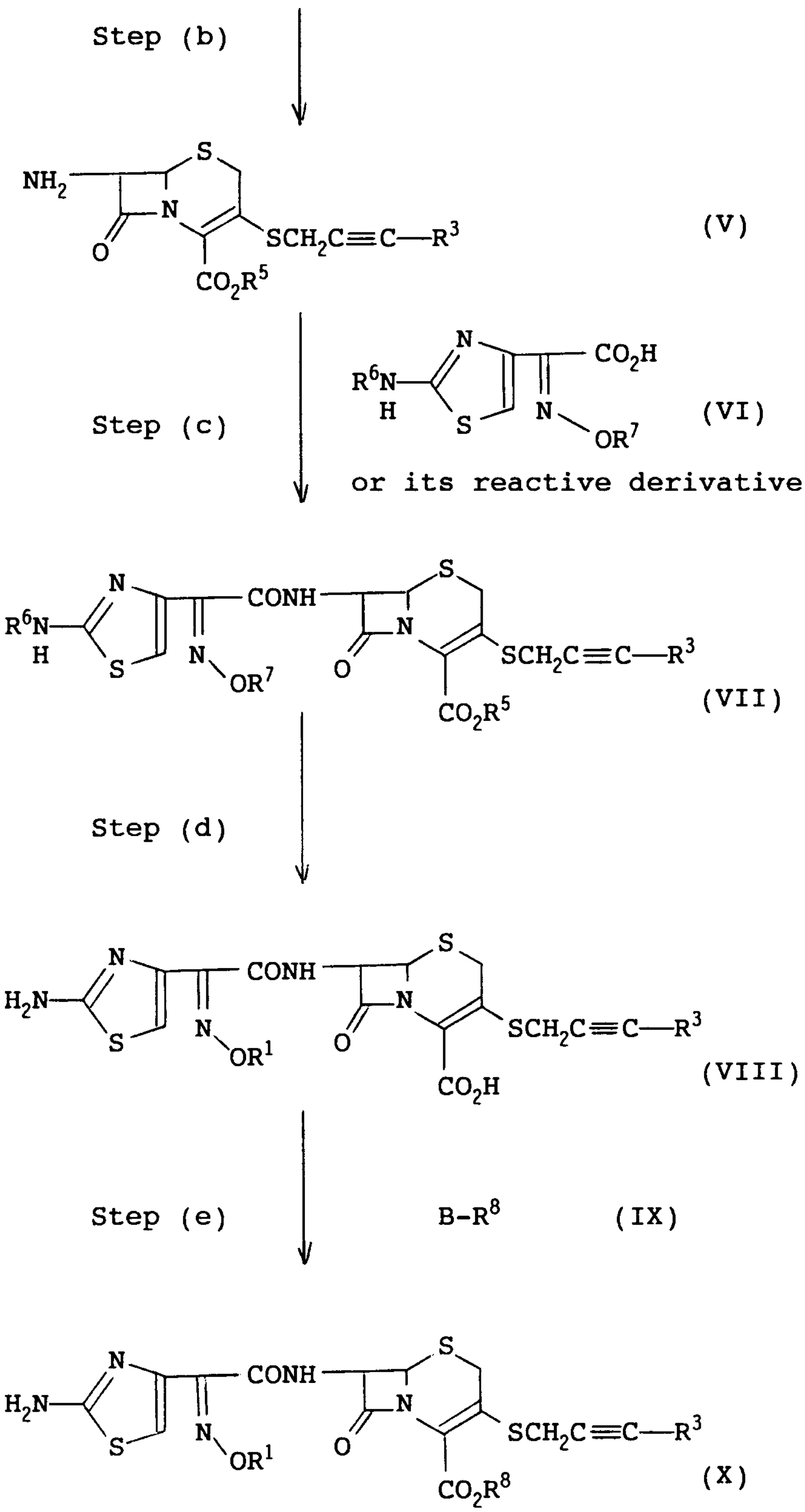


(V)

(VI)

(VII)

(VIII)

(IX)

(X)

[In Preparation Method I, $R^1$ and $R^3$ are as defined above, $R^4$ is a protecting group of the amino group such as a phenylacetyl group, a phenoxyacetyl group, a trityl group, a phthaloyl group, a formyl group or a benzoyl group.

$R^5$ is a protecting group of the carboxyl group such as a methyl group, an ethyl group, a propyl group, an isopropyl group, a t-butyl group, a vinyl group, an allyl group, an ethynyl group, a methoxymethyl group,

an ethoxymethyl group, a 1-methoxyethyl group, a methylthiomethyl group, a carboxymethyl group, a carboxyethyl group, a t-butoxycarbonylmethyl group, a 2-(t-butoxycarbonyl)ethyl group, a benzyl group, p-nitrobenzyl group, a bis-(4-methoxyphenyl)methyl group, a 3,4-dimethoxybenzyl group, a trityl group, a phenethyl group, a phenacyl group, a 2,2,2-trichloroethyl group, a trimethylsilyl group, a 4-hydroxy-3,5-di(t-butyl)benzyl group, a phenyl group, a tolyl group or a xylyl group.

$R^6$ is a protecting group of the amino group such as a monochloroacetyl group, a formyl group, a benzyloxycarbonyl group, a p-nitrobenzyloxycarbonyl group, a p-methoxybenzyloxycarbonyl group, a t-butoxycarbonyl group or a trimethylsilyl group or a hydrogen atom.

$R^7$ is a protecting group of the hydroxyl group such as a formyl group, an acetyl group, a propionyl group, a methoxycarbonyl group, a butoxycarbonyl group, a t-butoxycarbonyl group, a benzoyl group, a toluoyl group, a benzenesulfonyl group, a tosyl group, a phenyl group, a 4-methoxybenzyl group, a 2,4-dimethoxybenzyl group, a trityl group or a tetrahydropyranyl group, or a lower alkyl group.

$R^8$ is the in vivo hydrolyzable group as described above. W, A and B are each a leaving group such as a halogen atom (e.g. a chlorine atom, a bromine atom or an iodine atom), a methanesulfonyloxy group, a trifluoromethanesulfonyloxy group, a diphenylphosphoryloxy group or a p-toluenesulfonyloxy group.]

Preparation Steps (a) to (e) are illustrated in more detail as follows:

Step (a): A known compound of Formula (II) is dissolved in a reaction-inert organic solvent, and the solution together with 1.0 to 1.2 molar equivalents of sodium hydrosulfide in the presence of a base is stirred to give a 3-mercapto form. The reaction temperature is from -50 to 100 °C, preferably from -40 to 5 °C. The reaction time is from 10 minutes to 4 hours, preferably from 10 minutes to an hour.

To the 3-mercapto form obtained in the above procedure, in the same reaction system without isolation, is added 1.0 to 2.0 molar equivalents of a compound of Formula (III) in the presence of a base with stirring at a reaction temperature of from -50 to 100 °C, preferably from -25 to 50 °C to give a 3-thio substituted form of Formula (IV).

The reaction time depends on the kind of the base to be used, the kind of the compound of Formula (III) to be used and the reaction temperature, but it is from 10 minutes to 5 hours, and usually from 10 minutes to 2 hours.

Preferred examples of the organic solvent in the above are N,N-dimethylformamide, N,N-dimethylacetamide, dimethyl sulfoxide, hexamethylphosphoric triamide, acetonitrile, tetrahydrofuran and dichloromethane, and they can be used alone or in admixture.

Preferred examples of the base are organic bases such as diisopropylethylamine, N,N-dimethylaminopyridine, N,N-dimethylaniline and triethylamine. The most preferred amount of the base is from 1.0 to 2.0 molar equivalents relative to the compound of Formula (II).

The 3-mercapto form obtained in the above procedure, after isolation, can be also used for the following reaction. That is, the 3-mercapto form is reacted with a compound of Formula (III) in the presence of a base and an organic solvent both of which are the same as those used above to give a 3-thio substituted form of Formula (IV). The reaction conditions are also the same as above.

Step (b): The protecting group of the amino group at the 7-position of the compound of Formula (IV) obtained in Step (a) is eliminated by a method frequently used in the field of the β-lactam chemistry to give a compound of Formula (V). For example, a compound of Formula (IV) wherein the protecting group $R^4$ is a phenoxyacetyl group, a phenylacetyl group or a benzoyl group is dissolved in dichloromethane or benzene, and 1.5 to 2.0 molar equivalents of phosphorus pentachloride and 2.0 to 3.0 molar equivalents of pyridine are added, and then the mixture is stirred at - 40 to 30 °C for 30 minutes to 3 hours. Subsequently, a large excess amount of methanol or isobutyl alcohol is added at -50 to 20 °C, and the mixture is stirred for 30 minutes to 2 hours. After the addition of a large excess amount of water, the mixture is stirred at -50 to 20 °C for 30 minutes to one hour to give a compound of Formula (V).

A compound of Formula (IV) wherein the protecting group $R^4$ is a trityl group is dissolved in a reaction-inert solvent (e.g. ethyl acetate), 1.0 to 1.5 molar equivalents of p-toluenesulfonic acid monohydrate is added under ice-cooling, followed by stirring for 1 to 5 hours to give a compound of Formula (V) in the form of p-toluenesulfonic acid salt. If necessary, the p-toluenesulfonic acid salt is treated with a base to give the compound of Formula (V) in a free form.

Step (c): In order to obtain the compound of Formula (VII) from the compound of Formula (V), the compound of Formula (V) is reacted with a 2-aminothiazoleacetic acid derivative of Formula (VI) in the presence of a condensing agent or reacted with a reactive derivative of the compound of Formula (VI). Examples of the condensing agent are N,N-dicyclohexylcarbodiimide, 1-ethoxycarbonyl-2-ethoxy-1,2-dihydroquinoline, carbonyldiimidazole, diphenylphosphoryl azide and Vilsmeier reagent.

Examples of the reactive derivative of the compound of Formula (VI) are an acid halide (e.g. acid chloride or acid bromide of the compound of Formula (VI)), an acid anhydride (e.g. a symmetrical acid

anhydride of the compound of Formula (VI) or a mixed acid anhydride of the compound of Formula (VI) with ethyl chlorocarbonate, diphenylphosphoric acid or methanesulfonic acid) and an activated ester (e.g. an ester of p-nitrophenol, thiophenol, N-hydroxysuccinimide or 1-oxybenzotriazole).

When an acid chloride is used as a reactive derivative of the compound of Formula (VI), first the compound of Formula (VI) is dissolved in a reaction-inert solvent. To the solution is added 1.0 to 1.1 molar equivalents of phosphorus pentachloride in the presence of a base at a -30 to -10 °C, followed by stirring for 10 to 30 minutes to prepare an acid chloride of the compound of Formula (VI). To this is added a solution of 0.7 to 1.0 molar equivalent of the compound of Formula (V) in the same reaction-inert solvent as described above at a temperature ranging from -30 to 0 °C, followed by stirring for 10 to 30 minutes to give a compound of Formula (VII). Preferred examples of the solvent in this step are dichloromethane, chloroform, acetonitrile and N,N-dimethylformamide. Preferred examples of the base are pyridine, triethylamine, N,N-dimethylaminopyridine, N,N-dimethylaniline and diisopropylethylamine. The amount of the base to be used is 4.0 to 5.5 molar equivalents relative to the compound of Formula (V).

Referring to a mixed acid anhydride of the compound of Formula (VI) with phosphorus oxychloride as an example of the reactive derivative of the compound of Formula (VI), first the compound of Formula (V) and 1.0 to 1.3 molar equivalents of the compound of Formula (VI) are dissolved in the same reaction-inert solvent as described above. To the solution are added 2.0 to 4.0 molar equivalents of a base and 1.0 to 3.0 molar equivalents of phosphorus oxychloride at -20 to -5 °C, followed by stirring at a temperature ranging from -20 to 0 °C for 10 to 30 minutes to give a compound of Formula (VII). Preferred examples of the solvent in this step are ethyleneglycol dimethyl ether, tetrahydrofuran, dioxane, dichloromethane and chloroform. Preferred examples of the base are pyridine, triethylamine and diisopropylethylamine.

Referring to a mixed acid anhydride of the compound of Formula (VI) with methanesulfonic acid as an example of the reactive derivative of the compound of Formula (VI), first the compound of Formula (VI) is dissolved in a reaction-inert solvent. To the solution is added 1.0 to 1.1 molar equivalents of methanesulfonyl chloride in the presence of a base at -70 to -30 °C, followed by stirring for 20 to 40 minutes to prepare a mixed acid anhydride of the compound of Formula (VI). To this is added a solution of 0.5 to 0.7 molar equivalent of the compound of Formula (V) in the presence of base at a temperature ranging from -70 to - 30 °C, followed by stirring for 20 to 40 minutes to give a compound of Formula (VII). Preferred examples of the solvent in this step are N,N-dimethylformamide, tetrahydrofuran, acetonitrile, dichloromethane and chloroform. Preferred examples of the base are diisopropylethylamine, triethylamine, N,N-dimethyaniline, N,N-dimethylaminopyridine and pyridine. The amount of the base to be used is 1.0 to 2.2 molar equivalents relative to the compound of Formula (V).

Referring to an activated ester of the compound of Formula (VI) with 1-oxybenzotriazole as an example of the reactive derivative of the compound of Formula (VI), first the compound of Formula (V) is dissolved in a reaction-inert solvent. To the solution is added 1.0 to 1.1 molar equivalents of an activated ester form at 0 to 40 °C, followed by stirring for 5 to 20 hours to give a compound of Formula (VII). Preferred examples of the solvent in this step are N,N-dimethylformamide, tetrahydrofuran, acetonitrile, dichloromethane and chloroform.

Step (d): In order to obtain the compound of Formula (VIII) from the compound of Formula (VII) obtained in the above Step (c), the protecting groups of the compound of Formula (VII) are eliminated by hydrolysis or reduction frequently used in the field of the $\beta$-lactam chemistry.

(1) Hydrolysis

Hydrolysis is preferably carried out in the presence of an acid such as, for example, an inorganic acid (e.g. hydrochloric acid, hydrobromic acid or sulfuric acid), an organic acid (e.g. formic acid, acetic acid, trifluoroacetic acid, methanesulfonic acid, benzenesulfonic acid or p-toluenesulfonic acid). A Lewis acid such as boron trifluoride, boron trifluoride etherate, aluminium chloride, antimony pentachloride, ferric chloride, tin chloride, titanium tetrachloride or zinc chloride, or an acidic ion exchange resin can be used instead of the acid as described above. Furthermore, when the above organic acid or Lewis acid is used, hydrolysis is preferably carried out in the presence of a cation trapping agent such as anisole. Examples of the preferred solvent in hydrolysis are water, methanol, ethanol, tetrahydrofuran, N,N-dimethylformamide, dioxane, dichloromethane and nitromethane. When the acid described above is liquid, it can itself be used as a solvent.

(2) Reduction

Reduction may be carried out by a method of chemical reduction or catalytic reduction. A reducing agent to be used in the chemical reduction may be a combination of a metal such as zinc or iron with an organic or inorganic acid such as formic acid, acetic acid, trifluoroacetic acid, hydrochloric acid or hydrobromic acid. Examples of the catalyst to be used in the catalytic reduction are platinum black, platinum oxide, palladium black, palladium on carbon and Raney nickel.

Usually, the reduction may be carried out in a conventional solvent which is unaffected disadvantageously to the reaction such as water, methanol, ethanol, acetic acid, tetrahydrofuran, N,N-dimethylformamide, dioxane or a mixture thereof.

The reaction temperature of the above hydrolysis or reduction is not restricted, but usually the reaction may be carried out under cooling or warming.

Step (e): The compound of Formula (X) can be obtained by a reaction of the compound of Formula (VIII) with the compound of Formula (IX) in the presence of a base or a reaction of a salt of the compound of Formula (VIII) with the compound of Formula (IX). Preferred examples of the base in this step are an organic base such as triethylamine, diisopropylethylamine or N,N-dimethylaminopyridine, and an inorganic base such as sodium bicarbonate, sodium carbonate, cesium carbonate, sodium hydroxide or potassium hydroxide. Examples of the salt of the compound of Formula (VIII) are salts with metals such as silver, sodium, potassium, calcium or magnesium. Preferring to the reaction using the sodium salt as an example of the salt of the compound of Formula (VIII), sodium salt of the compound of Formula (VIII) is dissolved or suspended in a reaction-inert solvent, and then 1.0 to 2.0 molar equivalents of the compound of Formula (IX) is added, followed by stirring at -50 to 50 °C, preferably at -30 to 20 °C, for 5 minutes to 2 hours, preferably for 10 minutes to an hour. Preferred examples of the solvent in this step are acetone, chloroform, dichloromethane, tetrahydrofuran, acetonitrile, diethyl ether, methanol, ethanol, benzene, N,N-dimethylformamide, N,N-dimethylacetamide, dimethyl sulfoxide, hexamethylphosphoric triamide and water.

The compound of Formula (X) can be also prepared according to Preparation Method II.

Preparation Method (II)

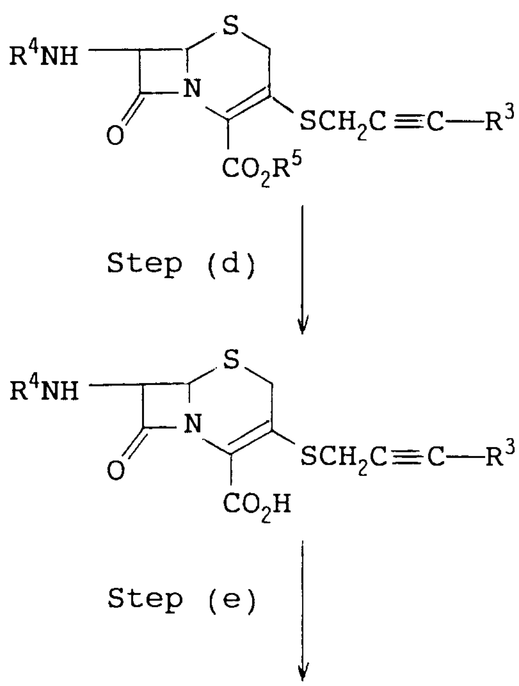


(IV)

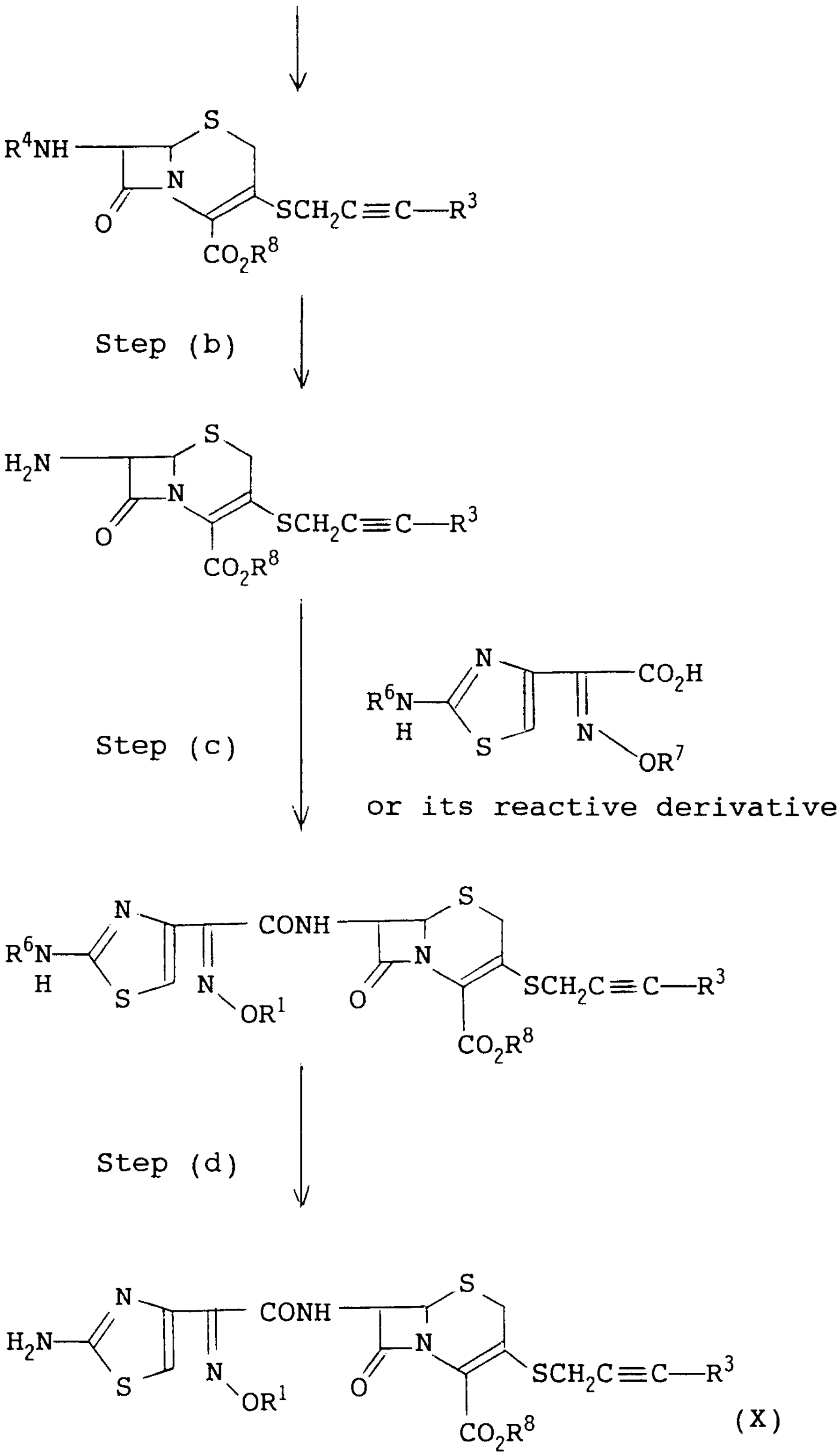


(X)

[In the reaction route of Preparation Method II, $R^3$ to $R^8$ and Steps (b) to (e) are as defined above].

## BEST MODE FOR CARRYING OUT THE INVENTION

The present invention is illustrated in more detail by the following examples.

### Example 1

(a) To a solution of 3.0 g (5.6 mM) of p-methoxybenzyl 7β-phenylacetamido-3-methanesulfonyloxy-3-cephem-4-carboxylate in 20 ml of N,N-dimethylformamide were added at -10°C a solution of 630 mg (6.7 mM) of 70% sodium hydrosulfide in 10 ml of N,N-dimethylformamide and 1.1 g (8.4 mM) of diisopropylethylamine. The mixture was stirred at the same temperature for 30 minutes, and 1.34 g (12.2 mM) of propargyl bromide was added thereto, followed by further stirring under ice-cooling for an hour. After the reaction, 100 ml of 0.5% hydrochloric acid was added, the mixture was extracted with 200 ml of ethyl acetate, washed twice with 100 ml each of a saturated aqueous sodium chloride solution and dried over anhydrous magnesium sulfate. The solvent was evaporated, and the residue was chromatographed on silica gel column (eluent ; ethyl acetate : dichloromethane = 1:2) to give 1.8 g of p-methoxybenzyl 7β-phenylacetamido-3-(2-propynyl)thio-3-cephem-4-carboxylate.

NMR (DMSO-$d_6$) δ (ppm):

3.28 (1H, t, J=2.5Hz), 3.49 and 3.57 (2H, ABq, J=14Hz), 3.73 (1H, dd, J=2.5, 17Hz), 3.75 (3H, S), 3.82 (1H, dd, J=2.5, 17Hz), 3.83 and 3.90 (2H, ABq, J=17Hz), 5.12 and 5.21 (2H, ABq, J=12Hz), 5.14 (1H, d, J=4.5Hz), 5.64 (1H, dd, J=4.5, 8.5Hz), 6.92 (2H, d, J=8.5Hz), 7.17 ~ 7.39 (7H, m), 9.13 (1H, d, J=8.5Hz)

$$IR\ \nu_{max}^{KBr} cm^{-1};$$

3269, 3031, 2958, 1780, 1698, 1655, 1614, 1537, 1517, 1497, 1455, 1391, 1360, 1288, 1247, 1228, 1173, 1119, 1090, 1032

(b) To a solution of 1.65 g (3.2 mM) of p-methoxybenzyl 7β-phenylacetamido-3-(2-propynyl)thio-3-cephem-4-carboxylate obtained in the above (a) in 20 ml of anhydrous dichloromethane were added 0.51 g (6.5 mM) of pyridine and 1.02 g (4.9 mM) of phosphorus pentachloride under ice-cooling, followed by stirring at room temperature for 1.5 hours. 1.45 g (19.6 mM) of isobutyl alcohol was added under ice-cooling, and the mixture was stirred at the same temperature for an hour. Five ml of water was added, and the mixture was stirred for a further 30 minutes. After the reaction, the organic layer of the reaction solution was washed with 5 ml of a saturated aqueous sodium chloride solution and dried over anhydrous magnesium sulfate. The solvent was concentrated (about 5 ml), and the residue was added dropwise to 80 ml of diisopropyl ether. The precipitated powder was collected by filtration to give 1.0 g of p-methoxybenzyl 7β-amino-3-(2-propynyl)thio-3-cephem-4-carboxylate hydrochloric acid salt.

NMR (DMSO-$d_6$) δ (ppm);

3.34 (1H, t, J=2.5Hz), 3.75 (3H, S), 3.82 and 3.97 (2H, ABq, J=16.5Hz), 3.89 (2H, d, J=2.5Hz), 5.07 (1H, d, J=4.5Hz), 5.17 (2H, bS), 5.25 (1H, d, J=4.5Hz), 6.92 (2H, d, J=8.5Hz), 7.34 (2H, d, J=8.5Hz), 9.23 (3H, bS)

$$IR\ \nu_{max}^{KBr} cm^{-1};$$

3436, 3271, 2898, 2649, 1772, 1694, 1613, 1586, 1516, 1467, 1401, 1367, 1291, 1215, 1176, 1134, 1116, 1030

(c) To a cooled (-20°C) solution of 980 mg (2.3 mM) of p-methoxybenzyl 7β-amino-3-(2-propynyl)thio-3-cephem-4-carboxylate hydrochloric acid salt obtained in the above (b) and 1.75 g (2.5 mM) of 2-(tritylaminothiazol-4-yl)-2-[(Z)-trityloxyimino]acetic acid sodium salt in 30 ml of tetrahydrofuran were added 546 mg (6.9 mM) of pyridine and 705 mg (4.6 mM) of phosphorus oxychloride, followed by stirring at -15 to -5°C for 20 minutes. After the reaction, 50 ml of water was added, the mixture was extracted with 100 ml of ethyl acetate, washed with 50 ml of a saturated aqueous sodium chloride solution and then dried over anhydrous magnesium sulfate. The solvent was evaporated, and the residue was chromatographed on silica gel column (eluent; ethyl acetate : n-hexane = 1:2) to give 1.82 g of p-methoxybenzyl 7β-{2-(2-tritylaminothiazol-4-yl)-2-[(Z)-trityloxyimino]acetamido}-3-(2-propynyl)thio-3-cephem-4-carboxylate.

NMR ($DMSO-d_6$) δ (ppm);

3.28 (1H, t, J=2.5Hz), 3.75 (3H, S), 3.80 (2H, d, J=2.5Hz), 3.78 and 3.88 (2H, ABq, J=17Hz), 5.19 (2H, bS), 5.25 (1H, d, J=4.5Hz), 5.77 (1H, dd, J=4.5, 8.5Hz), 6.66 (1H, S), 6.93 (2H, d, J=8.5Hz), 7.06 ~ 7.42 (32H, m), 8.77 (1H, bS), 9.85 (1H, d, J=8.5Hz)

$$IR\ \nu_{max}^{KBr} cm^{-1};$$

3286, 3057, 1786, 1729, 1688, 1613, 1516, 1493, 1448, 1386, 1360, 1249, 1219, 1175, 1116, 1085, 1034

(d) To a mixture of 3.1 ml of trifluoroacetic acid, 0.62 ml of anisole and 6.2 ml of dichloromethane was added under ice-cooling 1.77 g (1.7 mM) of p-methoxybenzyl 7β-{2-(2-tritylaminothiazol-4-yl)-2-[(Z)-trityloxyimino]acetamido}-3-(2-propynyl)thio-3-cephem-4-carboxylate obtained in the above (c), followed by stirring for 1.5 hours. The reaction solution was added dropwise to 64 ml of diisopropyl ether, and the precipitated crystals were collected by filtration. The crystals were dissolved in 6.2 ml of formic acid, followed by stirring at room temperature for an hour. The insoluble substances were removed by filtration, and the filtrate was added dropwise to 77 ml of diisopropyl ether. The precipitated crystals were collected by filtration to give 650 mg of 7β-{2-(2-aminothiazol-4-yl)-2-[(Z)-hydroxyimino]acetamido}-3-(2-propynyl)thio-3-cephem-4-carboxylic acid formic acid salt. The formic acid salt was suspended in 10 ml of water, the suspension was dissolved by adding 356 mg (4.2 mM) of sodium bicarbonate. The solution was chromatographed on Toyopal (HW-40F) column (eluent; water) to give 500 mg of 7β-{2-(2-aminothiazol-4-yl)-2-[(Z)-hydroxyimino]-acetamido}-3-(2-propynyl)thio-3-cephem-4-carboxylic acid sodium salt.

NMR ($DMSO-d_6$) δ (ppm);

3.13 (1H, t, J=2.5Hz), 3.47 (1H, dd, J=2.5, 16Hz), 3.36 (1H, dd, J=2.5, 16Hz), 3.56 and 3.67 (2H, ABq, J=17Hz), 4.98 (1H, d, J=4.5Hz), 5.59 (1H, dd, J=4.5, 8Hz), 6.65 (1H, S), 7.12 (2H, bS), 9.41 (1H, d, J=8Hz), 11.30 (1H, S)

$$IR\ \nu_{max}^{KBr} cm^{-1};$$

3283, 1762, 1662, 1608, 1533, 1395, 1353, 1183, 1046

Example 2

To a solution of 420 mg (0.9 mM) of the sodium salt obtained in the above Example 1(d) in 7 ml of N,N-dimethylformamide was added 287 mg (1.2 mM) of pivaloyloxymethyl iodide under ice-cooling, followed by stirring for an hour. After the reaction, 30 ml of water was added, and the mixture was extracted with 50 ml of ethyl acetate, washed with 30 ml of a saturated aqueous sodium chloride solution and dried over anhydrous magnesium sulfate. The solvent was evaporated, and the residue was chromatographed on Sephadex LH-20 column (eluent; acetone) to give 255 mg of pivaloyloxymethyl 7β-{2-(2-aminothiazol-4-yl)-2-[(Z)-hydroxyimino]acetamido}-3-(2-propynyl)thio-3-cephem-4-carboxylate.

NMR ($DMSO-d_6$) δ (ppm);

1.16 (9H, S), 3.30 (1H, t, J=2.5Hz), 3.80 (1H, dd, J=2.5, 17Hz), 3.83 and 3.91 (2H, ABq, J=17Hz), 3.87 (1H, dd, J=2.5, 17Hz), 5.22 (1H, d, J=4.5Hz), 5.75 (1H, dd, 4.5, 8Hz), 5.78 and 5.90 (2H, ABq, J=6Hz), 6.69 (1H, S), 7.14 (2H, bS), 9.48 (1H, d, J=8Hz), 11.31 (1H, S)

$$IR\ \nu_{max}^{KBr} cm^{-1};$$

3435, 2975, 1781, 1754, 1674, 1616, 1531, 1481, 1460, 1371, 1283, 1217, 1158, 1131, 1110, 1029

Example 3

To a solution of 280 mg (0.51 mM) of the pivaloyloxymethyl ester obtained in the above Example 2 in 1 ml of acetone was added 110 mg (0.58 mM) of p-toluenesulfonic acid monohydrate, followed by stirring at room temperature for 10 minutes. After the reaction, the reaction solution was added dropwise to 10 ml of diisopropyl ether, and the precipitated crystals were collected by filtration to give 230 mg of pivaloyloxymethyl 7β-{2-(2-aminothiazol-4-yl)-2-[(Z)-hydroxyimino]-acetamido}-3-(2-propynyl)thio-3-cephem-4-carboxylate mono-p-toluenesulfonic acid salt.

NMR (DMSO-$d_6$) δ (ppm);

1.16 (9H, S), 2.29 (3H, S), 3.31 (1H, t, J = 2.5Hz), 3.83 (1H, dd, J = 2.5, 7Hz), 3.87 (1H, dd, J = 2.5, 7Hz), 3.88 (2H, bS), 5.25 (1H, d, J = 4.5Hz), 5.75 (1H, dd, J = 4.5, 8Hz), 5.78 and 5.90 (2H, ABq, J = 6Hz), 6.87 (1H, S), 7.12 (2H, d, J = 8Hz), 7.47 (2H, d, J = 8Hz), 8.51 (3H, bS), 9.70 (1H, d, J = 8Hz), 12.10 (1H, S)

$$IR\ \nu_{max}^{KBr} cm^{-1};$$

3412, 2975, 1785, 1756, 1641, 1530, 1481, 1459, 1372, 1280, 1214, 1161, 1125, 1110, 1034, 1010

Example 4

To a solution of 280 mg (0.51 mM) of the pivaloyloxymethyl ester obtained in the above Example 2 in 0.7 ml of acetonitrile was added a solution of 62 mg (0.53 mM) of maleic acid in 2.3 ml of acetonitrile, followed by standing at room temperature for 2 minutes. After addition of 3 ml of diisopropyl ether, the mixture was allowed to stand for an hour, and the precipitated crystals were collected by filtration to give 250 mg of pivaloyloxymethyl 7β-{2-(2-aminothiazol-4-yl)-2-[(Z)-hydroxyimino]acetamido}-3-(2-propynyl)thio-3-cephem-4-carboxylate mono-maleic acid salt.

NMR (DMSO-$d_6$) δ (ppm);

1.16 (9H, S), 3.30 (1H, t, J = 2.5Hz), 3.75 ~ 3.95 (4H, m), 5.22 (1H, d, J = 4.5Hz), 5.75 (1H, dd, J = 4.5, 8Hz), 5.78 and 5.90 (2H, ABq, J = 6Hz), 6.25 (2H, S), 6.71 (1H, S), 7.28 (2H, bS), 9.50 (1H, d, J = 8Hz), 11.40 (1H, S)

$$IR\ \nu_{max}^{KBr} cm^{-1};$$

3284, 2976, 1784, 1756, 1662, 1627, 1531, 1365, 1110

Example 5

To a solution of 500 mg (1.1 mM) of the sodium salt obtained in the above Example 1(d) in 5 ml of N,N-dimethylformamide was added 392 mg (1.5 mM) of 1-iodoethylisopropyl carbonate under ice-cooling, followed by stirring for 45 minutes. After the reaction, 30 ml of water was added, and the mixture was extracted with 50 ml of ethyl acetate, washed with 30 ml of a saturated aqueous sodium chloride solution and dried over anhydrous magnesium sulfate. The solvent was evaporated, and the residue was chromatographed on Sephadex LH-20 column (eluent; acetone) to give 230 mg of 1-(isopropyloxycarbonyloxy)ethyl 7β-{2-(2-aminothiazol-4-yl)-2-[(Z)-hydroxyimino]acetamido}-3-(2-propynyl)thio-3-cephem-4-carboxylate. To the ester was added 1.5 ml of acetone, followed by stirring at room temperature for an hour. The precipitated crystals were collected by filtration to give 97 mg of one of the diastereomer isomers.

NMR (DMSO-$d_6$) δ (ppm);

1.23 (3H, d, J = 6Hz), 1.25 (3H, d, J = 6Hz), 1.48 (3H, d, J = 6Hz), 3.31 (1H, bS), 3.82 (1H, d, J = 17Hz), 3.85 (2H, bS), 3.92 (1H, d, J = 17Hz), 4.78 (1H, m), 5.20 (1H, d, J = 5Hz), 5.73 (1H, d, J = 5, 9Hz), 6.69 (1H, S), 6.80 (1H, q, J = 6Hz), 7.13 (2H, bS), 9.47 (1H, d, J = 9Hz), 11.30 (1H, S)

$$IR\ \nu_{max}^{KBr} cm^{-1};$$

3293, 2986, 1785, 1760, 1697, 1621, 1531, 1448, 1378, 1354, 1294, 1267, 1226, 1163, 1122, 1101, 1074, 1043

Example 6

The filtrate obtained in the filtration by which the precipitated crystals were obtained in the above Example 5 was concentrated to give 133 mg of another diasteromer isomer of that of Example 5 (containing about 25% of the isomer of Example 5).

NMR (DMSO-$d_6$) $\delta$ (ppm);

1.24 (6H, d, J = 6Hz), 1.48 (3H, d, J = 5.5Hz), 3.31 (1H, bS), 3.85 and 3.93 (2H, ABq, J = 17Hz), 3.86 (2H, bS), 4.80 (1H, m), 5.22 (1H, d, J = 5Hz), 5.78 (1H, dd, J = 5, 8.5Hz), 6.68 (1H, S), 6.76 (1H, q, J = 5.5Hz), 7.13 (2H, bS), 9.48 (1H, d, J = 8.5Hz), 11.32 (1H, S)

$$IR\ \nu_{max}^{KBr} cm^{-1};$$

3290, 2983, 1762, 1672, 1616, 1531, 1377, 1271, 1218, 1167, 1101, 1074, 1044

Example 7

To a solution of 216 mg (0.38 mM) of the diastereomer isomer obtained in Example 5 in 10 ml of acetone was added a solution of 48 mg (0.42 mM) of maleic acid in methanol, and the mixture was concentrated to about 3 ml at room temperature. To the concentrated solution was added 5 ml of diisopropyl ether, followed by standing for an hour. The precipitated crystals were collected by filtration to give 203 mg of 1-(isopropyloxycarbonyloxy)ethyl 7$\beta$-{2-(2-aminothiazol-4-yl)-2-[(Z)-hydroxyimino]-acetamido}-3-(2-propynyl)thio-3-cephem-4-carboxylate harf-maleic acid salt.

NMR (DMSO-$d_6$) $\delta$ (ppm);

1.23 (3H, d, J = 6Hz), 1.25 (3H, d, J = 6Hz), 1.48 (3H, d, J = 5.5Hz), 3.31 (1H, t, J = 2.5Hz), 3.83 and 3.91 (2H, ABq, J = 17Hz), 3.85 (2H, d, J = 2.5Hz), 5.20 (1H, d, J = 4.5Hz), 5.73 (1H, dd, J = 4.5, 8Hz), 6.24 (1H, S), 6.70 (1H, S), 6.81 (1H, q, J = 5.5Hz), 7.23 (3H, bS), 9.49 (1H, d, J = 8Hz), 11.37 (1H, S)

$$IR\ \nu_{max}^{KBr} cm^{-1};$$

3293, 2985, 1785, 1761, 1696, 1622, 1530, 1378, 1355, 1294, 1268, 1226, 1164, 1102, 1075, 1043

Example 8

(a) To a cooled (-20 ° C) solution of 700 mg (1.6 mM) of p-methoxybenzyl 7$\beta$-amino-3-(2-propynyl)thio-3-cephem-4-carboxylate hydrochloric acid salt obtained in Example 1(b) and 839 mg (1.8 mM) of 2-(tritylaminothiazol-4-yl)-2-[(Z)-methoxyimino]acetic acid sodium salt in 20 ml of tetrahydrofuran were added 390 mg (4.9 mM) of pyridine and 503 mg (3.3 mM) of phosphorus oxychloride, followed by stirring at -15 to -5 ° C for 20 minutes. After the reaction, 50 ml of water was added, the mixture was extracted with 100 ml of ethyl acetate, washed with 50 ml of a saturated aqueous sodium chloride solution and dried over anhydrous magnesium sulfate. The solvent was evaporated, and the residue was chromatographed on silica gel column (eluent; ethyl acetate : n-hexane = 1:1) to give 1.1 g of p-methoxybenzyl 7$\beta$-{2-(2-tritylaminothiazol-4-yl)-2-[(Z)-methoxyimino]acetamido}-3-(2-propynyl)thio-3-cephem-4-carboxylate.

NMR (DMSO-$d_6$) $\delta$ (ppm);

3.27 (1H, t, J = 2.5Hz), 3.65 ~ 3.92 (4H, m), 3.74 (3H, S), 3.81 (3H, S), 5.13 and 5.19 (2H, ABq, J = 12Hz), 5.16 (1H, d, J = 4.5Hz), 5.64 (1H, dd, J = 4.5, 8Hz), 6.74 (1H, S), 6.92 (2H, d, J = 8.5Hz), 7.17 ~ 7.42 (17H, m), 8.81 (1H, bS), 9.55 (1H, d, J = 8Hz)

$$IR\ \nu_{max}^{KBr} cm^{-1};$$

3328, 2937, 1781, 1730, 1683, 1613, 1516, 1448, 1386, 1362, 1285, 1248, 1220, 1174, 1117, 1037, 1003

(b) To a mixture of 3.0 ml of trifluoroacetic acid, 0.6 ml of anisole and 3.0 ml of dichloromethane was added under ice-cooling 1.0 g (1.2 mM) of p-methoxybenzyl 7β-{2-(2-tritylaminothiazol-4-yl)-2-[(Z)-methoxyimino]acetamido}-3-(2-propynyl)thio-3-cephem-4-carboxylate obtained in the above (a), followed by stirring under ice-cooling for an hour and at room temperature for 20 minutes. After the reaction, the reaction solution was added dropwise to 50 ml of diisopropyl ether, and the precipitated crystals were collected by filtration to give 580 mg of 7β-{2-(2-aminothiazol-4-yl)-2-[(Z)-methoxyimino]acetamido}-3-propargylthio-3-cephem-4-carboxylic acid trifluoroacetic acid salt. The trifluoroacetic acid salt was suspended in 5 ml of water, and the suspension was dissolved by adding 257 mg (3.1 mM) of sodium bicarbonate. The solution was chromatographed on Toyopal (HW-40F) column (eluent; water) to give 454 mg of 7β-{2-(2-aminothiazol-4-yl)-2-[(Z)-methoxyimino]acetamido}-3-(2-propynyl)thio-3-cephem-4-carboxylic acid sodium salt.

NMR (DMSO-$d_6$) δ (ppm); 3.12 (1H, t, J = 2.5Hz), 3.47 (1H, dd, J = 2.5, 16Hz), 3.54 and 3.68 (2H, ABq, J = 17Hz), 3.63 (1H, dd, J = 2.5, 16Hz), 3.83 (3H, S), 4.98 (1H, d, J = 5Hz), 5.57 (1H, dd, J = 5, 8.5Hz), 6.73 (1H, S), 7.12 (2H, bS), 9.54 (1H, d, J = 8.5Hz)

$$IR\ \nu_{max}^{KBr} cm^{-1};$$

3401, 3274, 1762, 1657, 1607, 1535, 1391, 1353, 1181, 1128, 1040

Example 9

To a solution of 400 mg (0.8 mM) of the sodium salt obtained in the above Example 8(b) in 6.0 ml of N,N-dimethylformamide was added 265 mg (1.1 mM) of pivaloyloxymethyl iodide under ice-cooling, followed by stirring for an hour. After the reaction, 30 ml of water was added, and the mixture was extracted with 50 ml of ethyl acetate, washed with 30 ml of a saturated aqueous sodium chloride solution and dried over anhydrous magnesium sulfate. The solvent was evaporated, and the residue was chromatographed on Sephadex LH-20 column (eluent; acetone) to give 272 mg of pivaloyloxymethyl 7β-{2-(2-aminothiazol-4-yl)-2-[(Z)-methoxyimino]acetamido}-3-(2-propynyl)thio-3-cephem-4-carboxylate.

NMR (DMSO-$d_6$) δ (ppm);

1.16 (9H, S), 3.30 (1H, t, J = 2.5Hz), 3.80 (1H, dd, J = 2.5, 17Hz), 3.84 (3H, S), 3.85 and 3.92 (2H, ABq, J = 17Hz), 3.87 (1H, dd, J = 2.5, 17Hz), 5.22 (1H, d, J = 4.5Hz), 5.75 (1H, dd, J = 4.5, 8.5Hz), 5.78 and 5.90 (2H, ABq, J = 6Hz), 6.78 (1H, S), 7.21 (2H, S), 9.61 (1H, d, J = 8.5Hz)

$$IR\ \nu_{max}^{KBr} cm^{-1};$$

3285, 2975, 1780, 1755, 1672, 1618, 1536, 1481, 1461, 1372, 1283, 1218, 1158, 1130, 1110, 1034

Following the procedures and reaction conditions similar to those of Example 1(a) to 1(d) and Example 2, the compounds of Example 10(a) to 10(d) and Example 11 were obtained.

Example 10(a)

p-Methoxybenzyl 7β-phenylacetamido-3-(2-butynyl)thio-3-cephem-4-carboxylate

NMR (DMSO-$d_6$) δ (ppm);

1.78 (3H, t, J = 2.5Hz), 3.50 and 3.57 (2H, ABq, J = 14Hz), 3.66 ~ 3.77 (2H, m), 3.75 (3H, S), 3.85 (2H, bS), 5.12 and 5.20 (2H, ABq, J = 12Hz), 5.13 (1H, d, J = 4.5Hz), 5.62 (1H, dd, J = 4.5, 8.5Hz), 6.92 (2H, d, J = 8.5Hz), 7.17 ~ 7.38 (7H, m), 9.11 (1H, d, J = 8.5Hz)

$$IR\ \nu_{max}^{KBr} cm^{-1};$$

3263, 3046, 2962, 1780, 1702, 1659, 1615, 1539, 1518, 1455, 1395, 1360, 1288, 1250, 1231, 1174, 1119, 1033

Example 10(b)

p-Methoxybenzyl 7β-amino-3-(2-butynyl)thio-3-cephem-4-carboxylate hydrochloric acid salt

NMR ($DMSO-d_6$) δ (ppm);
1.80 (3H, t, J = 2.5Hz), 3.75 (3H, S), 3.76 ~ 3.87 (2H, m), 3.82 and 3.94 (2H, ABq, J = 16.5Hz), 5.07 (1H, d, J = 4.5Hz), 5.16 (2H, S), 5.24 (1H, d, J = 4.5Hz), 6.92 (2H, d, J = 8.5Hz), 7.34 (2H, d, J = 8.5Hz), 9.15 (3H, bS)

$$IR\ \nu_{max}^{KBr} cm^{-1};$$

3436, 2915, 2231, 1780, 1699, 1613, 1515, 1464, 1396, 1294, 1249, 1213, 1176, 1124, 1032

Example 10(c)

p-Methoxybenzyl 7β-{2-(2-tritylaminothiazol-4-yl)-2-[(Z)-trityloxyimino]acetamido}-3-(2-butynyl)thio3-cephem-4-carboxylate

NMR ($DMSO-d_6$) δ (ppm);
1.77 (3H, t, J = 2.5Hz), 3.68 ~ 3.79 (2H, m), 3.81 (2H, bS), 5.18 (2H, bS), 5.24 (1H, d, J = 4.5Hz), 5.75 (1H, dd, J = 4.5, 8Hz), 6.67 (1H, S), 6.93 (2H, d, J = 8.5Hz), 7.06 ~ 7.73 (32H, m), 8.76 (1H, bS), 9.84 (1H, d, J = 8Hz)

$$IR\ \nu_{max}^{KBr} cm^{-1};$$

3382, 3057, 2957, 1786, 1731, 1688, 1613, 1516, 1493, 1448, 1360, 1249, 1218, 1175, 1116, 1085, 1034

Example 10(d)

7β-{2-(2-aminothiazol-4-yl)-2-[(Z)-hydroxyimino]acetamido}-3-(2-butynyl)thio-3-cephem-4-carboxylic acid sodium salt

NMR ($DMSO-d_6$) δ (ppm);
1.78 (3H, t, J = 2.5Hz), 3.43 (1H, dq, J = 16, 2.5Hz), 3.51 and 3.66 (2H, ABq, J = 17Hz), 3.56 (1H, dq, J = 16, 2.5Hz), 4.99 (1H, d, J = 4.5Hz), 5.59 (1H, dd, J = 4.5, 8Hz), 6.65 (1H, S), 7.12 (2H, bS), 9.39 (1H, d, J = 8Hz), 11.40 (1H, S)

$$IR\ \nu_{max}^{KBr} cm^{-1};$$

3412, 1762, 1662, 1607, 1533, 1393, 1353, 1183, 1129, 1046

Example 11

Pivaloyloxymethyl 7β-{2-(2-aminothiazol-4-yl)-2-[(Z)-hydroxyimino]acetamido}-3-(2-butynyl)thio-3-cephem-4-carboxylate

NMR ($DMSO-d_6$) δ (ppm);
1.16 (9H, S), 1.80 (3H, t, J = 2.5Hz), 3.74 (1H, dq, J = 2.5, 16Hz), 3.83 (1H, dq, J = 2.5, 16Hz), 3.84 and

3.88 (2H, ABq, J = 17Hz), 5.21 (1H, d, J = 4.5Hz), 5.74 (1H, dd, J = 4.5, 8Hz), 5.77 and 5.90 (2H, ABq, J = 6Hz), 7.12 (2H, bS), 9.46 (1H, d, J = 8Hz)

$$IR\ \nu_{max}^{KBr} cm^{-1};$$

3436, 2975, 1780, 1757, 1674, 1615, 1531, 1481, 1456, 1372, 1282, 1217, 1158, 1110, 1029

Example 12

(a) To a solution of 20 g of p-methoxybenzyl 7β-phenylacetamido-3-(2-propynyl)thio-3-cephem-4-carboxylate obtained in Example 1(a) in 60 ml of dichloromethane were added 6 ml of anisole and 30 ml of trifluoroacetic acid under ice-cooling, followed by stirring for 1.5 hours. The reaction solution was added dropwise to 1 ℓ of diisopropyl ether, and the precipitated crystals were collected by filtration to give 14.5 g of 7β-phenylacetamido-3-(2-propynyl)thio-3-cephem-4-carboxylic acid.

NMR (DMSO-$d_6$) δ (ppm);

3.28 (1H, t, J = 2.5Hz), 3.50 and 3.58 (2H, ABq, J = 14Hz), 3.71 (1H, dd, J = 2.5, 17Hz), 3.79 (1H, dd, J = 2.5, 17Hz), 3.84 (2H, bS), 5.11 (1H, d, J = 4.5Hz), 5.61 (1H, dd, J = 4.5, 8.5Hz), 7.17 ~ 7.38 (5H, m), 9.11 (1H, d, J = 8.5Hz), 13.42 (1H, bS)

$$IR\ \nu_{max}^{KBr} cm^{-1};$$

3273, 3047, 1785, 1702, 1670, 1538, 1452, 1405, 1346, 1274, 1238, 1173, 1106

(b) To a solution of 2.0 g (5.2 mM) of 7β-phenylacetamido-3-(2-propynyl)thio-3-cephem-4-carboxylic acid obtained in the above (a) in 20 ml of N,N-dimethylformamide was added 386 mg (3.6 mM) of sodium carbonate at room temperature, followed by stirring for 20 minutes. Then, 1.63 g (6.8 mM) of pivaloyloxymethyl iodide was added under ice-cooling, and the mixture was stirred for 30 minutes. After the reaction, 40 ml of 0.1N hydrochloric acid was added, and the mixture was extracted with 100 ml of ethyl acetate, washed twice with 50 ml each of a saturated aqueous sodium chloride solution and dried over anhydrous magnesium sulfate. The solvent was evaporated, and the residue was chromatographed on silica gel column (eluent; ethyl acetate : n-hexane = 2:1) to give 2.4 g of pivaloyloxymethyl 7β-phenylacetamido-3-(2-propynyl)thio-3-cephem-4-carboxylate.

NMR(DMSO-$d_6$) δ (ppm);

1.16 (1H, S), 3.30 (1H, t, J = 2.5Hz), 3.49 and 3.58 (2H, ABq, J = 14Hz), 3.79 (1H, dd, J = 2.5, 16Hz), 3.87 (1H, dd, J = 2.5, 16Hz), 3.86 and 3.94 (2H, ABq, J = 17Hz), 5.23 (1H, d, J = 4.5Hz), 5.64 (1H, dd, J = 4.5, 8Hz), 5.79 and 5.89 (2H, ABq, J = 6Hz), 7.21 ~ 7.33 (5H, m), 9.12 (1H, d, J = 8Hz)

$$IR\ \nu_{max}^{KBr} cm^{-1};$$

3288, 2976, 1786, 1758, 1707, 1515, 1482, 1455, 1375, 1288, 1236, 1157, 1110, 1029

(c) To a solution of 2.0 g (4.0 mM) of pivaloyloxymethyl 7β-phenylacetamido-3-(2-propynyl)thio-3-cephem-4-carboxylate obtained in the above (b) in 20 ml of anhydrous dichloromethane were added 0.63 g (8.0 mM) of pyridine and 1.24 g (6.0 mM) of phosphorus pentachloride under ice-cooling, followed by stirring at room temperature for an hour. Then, 1.77 g (24 mM) of isobutyl alcohol was added under ice-cooling, and the mixture was stirred for an hour. 6.5 ml of water was added, and the mixture was stirred for 30 minutes. After the reaction, the precipitated crystals were collected by filtration to give 1.42 g of pivaloyloxymethyl 7β-amino-3-(2-propynyl)thio-3-cephem-4-carboxylate hydrochloric acid salt.

NMR (DMSO-$d_6$) δ (ppm);

1.15 (9H, S), 3.36 (1H, t, J = 2.5Hz), 3.85 and 4.01 (2H, ABq, J = 16.5Hz), 3.94 (2H, d, J = 2.5Hz), 5.09 (1H, d, J = 4.5Hz), 5.26 (1H, d, J = 4.5Hz), 5.76 and 5.91 (2H, ABq, J = 6Hz), 9.19 (2H, bS)

$$IR\ \nu_{max}^{KBr} cm^{-1};$$

3436, 3267, 2976, 2897, 2648, 2586, 1771, 1742, 1718, 1588, 1541, 1471, 1447, 1400, 1278, 1210, 1161, 1126, 1110, 1034, 1011

(d) To 1.0 g (2.6 mM) of the hydrochloric acid salt obtained above was added 30 ml of 1% aqueous sodium bicarbonate solution, followed by extraction with 30 ml of ethyl acetate. The organic layer was washed with 20 ml of a saturated aqueous sodium chloride solution and dried over anhydrous magnesium sulfate. Evaporation of the solvent gave 905 mg of pivaloyloxymethyl 7$\beta$-amino-3-(2-propynyl)thio-3-cephem-4-carboxylate. To a solution of the ester in 15 ml of tetrahydrofuran was added 1.4 g (2.6 mM) of 1-oxybenzotriazole 2-(2-aminothiazol-4-yl)-2-[(Z)-trityloxyimino]acetate at room temperature, followed by stirring for 15 hours. The reaction solution, after addition of 30 ml of water, was extracted with 40 ml of ethyl acetate, washed successively with 15 ml of 15 aqueous sodium bicarbonate solution and 30 ml of a saturated aqueous sodium chloride solution and dried over anhydrous magnesium sulfate. The solvent was evaporated, and the residue was chromatographed on silica gel column (eluent; ethyl acetate : n-hexane = 1:1) to give 1.7 g of pivaloyloxymethyl 7$\beta$-{2-(2-aminothiazol-4-yl)-2-[(Z)-trityloxyimino]acetamido}-3-(2-propynyl)thio-3-cephem-4-carboxylate.

NMR (DMSO-$d_6$) $\delta$ (ppm);

1.17 (9H, S), 3.30 (1H, t, J = 2.5Hz), 3.83 and 3.93 (2H, ABq, J = 17Hz), 3.86 (2H, d, J = 2.5Hz), 5.30 (1H, d, J = 4.5Hz), 5.80 and 5.92 (2H, ABq, J = 6Hz), 5.86 (1H, dd, J = 4.5, 8.5Hz), 6.67 (1H, S), 7.17 ~ 7.37 (17H, m), 9.86 (1H, d, J = 8.5Hz)

$$IR\ \nu_{max}^{KBr} cm^{-1};$$

3442, 3283, 3089, 2976, 1790, 1753, 1683, 1619, 1538, 1492, 1448, 1371, 1279, 1217, 1158, 1108, 1030

(e) 1.0 g (1.3 mM) of the pivaloyloxymethyl ester obtained in the above (d) was dissolved in 3 ml of 90% formic acid, and stirred at room temperature for an hour. The precipitated crystals were removed by filtration, and the filtrate, after addition of 50 ml of water, was extracted with 100 ml of ethyl acetate, washed 8 times with 50 ml each of water and dried over anhydrous magnesium sulfate. The solvent was evaporated, and the residue was chromatographed on Toyopal column (eluent; dichloromethane : acetone = 1:1) to give 0.5 g of pivaloyloxymethyl 7$\beta$-{2-(2-aminothiazol-4-yl)-2-[(Z)-hydroxyimino]-acetamido}-3-(2-propynyl)thio-3-cephem-4-carboxylate, which was identical with the pivaloyloxymethyl ester obtained in the above Example 2 (Preparation Method I) in terms of NMR and IR spectrum.

Example 13

(a) To a solution of 2.0 g (5.2 mM) of 7$\beta$-phenylacetamido-3-(2-propynyl)thio-3-cephem-4-carboxylic acid obtained in Example 1(a) in 20 ml of N,N-dimethylformamide was added 386 mg (3.6 mM) of sodium carbonate at room temperature, followed by stirring for 20 minutes. 1.7 g (6.7 mM) of 1-iodoethylisopropyl carbonate was added under cooling at -8 °C, and the mixture was stirred for an hour. After the reaction, 70 ml of water was added, and the mixture was extracted with 100 ml of ethyl acetate, washed with 70 ml of a saturated aqueous sodium chloride solution and dried over anhydrous magnesium sulfate. The solvent was evaporated, and 11 ml of methanol was added to the residue, followed by standing for an hour. The precipitated crystals were collected by filtration, and the resulting crystals were recrystallized from 10 ml of methanol to give 811 mg of a single diastereomer isomer of 1-(isopropyloxycarbonyloxy)ethyl 7$\beta$-phenylacetamido-3-(2-propynyl)thio-3-cephem-4-carboxylate.

NMR (DMSO-$d_6$) $\delta$ (ppm);

1.29 (3H, d, J = 6Hz), 1.30 (3H, d, J = 6Hz), 1.55 (3H, d, J = 5.5Hz), 2.33 (1H, t, J = 2.5Hz), 3.42 (1H, dd, J = 2.5, 18Hz), 3.58 (1H, dd, J = 2.5, 18Hz), 3.64 (2H, S), 3.66 and 3.72 (2H, ABq, J = 17Hz), 4.91 (1H, m), 4.97 (1H, d, J = 5Hz), 5.73 (1H, dd, J = 5, 9Hz), 6.21 (1H, d, J = 9Hz), 6.96 (1H, q, J = 5.5Hz), 7.20 ~ 7.42 (5H, m)

$$IR\ \nu_{max}^{KBr} cm^{-1};$$

3339, 3291, 2988, 1781, 1757, 1694, 1529, 1454, 1379, 1290, 1269, 1222, 1164, 1100, 1075, 1044, 1001

Following the procedures and reaction conditions similar to those disclosed in Example 12(c) to (e), the following compounds of Example 13(b) to (d) were obtained.

Example 13(b)

1-(Isopropyloxycarbonyloxy)ethyl 7β-amino-3-(2-propynyl)thio-3-cephem-4-carboxylate hydrochloric acid salt

NMR (DMSO-$d_6$) δ (ppm);

1.22 (3H, d, J = 6Hz), 1.24 (3H, d, J = 6Hz), 1.47 (3H, d, J = 5.5Hz), 3.37 (1H, t, J = 2.5Hz), 3.82 and 4.01 (2H, ABq, J = 16Hz), 3.92 (2H, d, J = 2.5Hz), 4.81 (1H, m), 5.05 (1H, d, J = 5Hz), 5.24 (1H, d, J = 5Hz), 6.75 (1H, q, J = 5.5Hz), 9.08 (2H, bS)

$$IR\ \nu_{max}^{KBr} cm^{-1};$$

3436, 3284, 2987, 2893, 1776, 1755, 1724, 1589, 1543, 1470, 1397, 1295, 1270, 1209, 1191, 1162, 1136, 1114, 1082, 1037

Example 13(c)

1-(Isopropyloxycarbonyloxy)ethyl 7β-{2-(2-aminothiazol-4-yl)-2-[(Z)-trityloxyimino]acetamido}-3-(2-propynyl)-thio-3-cephem-4-carboxylate

NMR (DMSO-$d_6$) δ (ppm);

1.21 (3H, d, J = 6Hz), 1.23 (3H, d, J = 6Hz), 1.50 (3H, d, J = 5.5Hz), 3.32t, J = 2.5Hz), 3.83 and 3.94 (2H, ABq, J = 17Hz), 3.88 (2H, d, J = 2.5Hz), 4.79 (1H, m), 5.29 (1H, d, J = 5Hz), 5.86 (1H, dd, J = 5, 8.5Hz), 6.69 (1H, S), 6.82 (1H, q, J = 5.5Hz), 7.20 ~ 7.42 (17H, m), 9.85 (1H, d, J = 8.5Hz)

$$IR\ \nu_{max}^{KBr} cm^{-1};$$

3442, 3285, 3089, 2984, 2936, 1790, 1762, 1683, 1619, 1538, 1493, 1448, 1376, 1268, 1218, 1161, 1102, 1074, 1043

Example 13(d)

1-(Isopropyloxycarbonyloxy)ethyl 7β-{2-(2-aminothiazol-4-yl)-2-[(Z)-hydroxyimino]acetamido}-3-(2-propynyl)thio-3-cephem-4-carboxylate.

This compound was identical with the ester obtained in Example 5 (Preparation Method II) in terms of NMR and IR spectrum.

INDUSTRIAL UTILIZATION

The compounds of Formula (I) and the non-toxic salts thereof of the present invention exhibit not only strong antibacterial activity against various pathogenic bacteria including gram-positive and gram-negative bacteria, but also good oral absorbability, and therefore, they are useful as antibacterial agents for oral administration.

For the purposes, the compounds of the present invention may be administered orally in dosage forms such as tablets, pills, capsules or granules, all of which can be prepared according to conventional pharmaceutical practices. In the above preparations can be used conventional additives such as fillers, binding agents, pH adjusting agents or solubilizers.

The dose of the compound of the present invention for a patient to be treated is varied depending on the age of the patient and the kind and conditions of the disease, but usually it is from 10 to 3000 mg,

which can be administered in a single dose or several divided doses per day.

Next, the results of the antibacterial activity test of the compounds of the present invention are shown below.

Experiment 1

The antibacterial test against various bacteria was carried out by the agar plate dilution method (inoculum size : $10^6$ cells/ml), and the results are shown in the following Table 1.

Table 1

| Bacteria \ Test drug | A | B | C | D |
|---|---|---|---|---|
| *Staphylococcus aureus* 209P-JP | 0.20 | 25 | 0.39 | 0.20 |
| *Staphylococcus epidermidis* sp-al-1 | 0.20 | 25 | 0.39 | 0.20 |
| *Enterococcus faecalis* CSJ 1212 | 1.56 | >100 | 1.56 | 3.13 |
| *Eschelichia coli* NIHJ JC-2 | 0.20 | 0.39 | 0.39 | 0.39 |
| *Klebsiella pneumoniae* IFO 3317 | 0.05 | 0.025 | 0.10 | 0.20 |
| *Proteus mirabilis* IFO 3849 | 0.10 | 0.025 | 0.20 | 0.20 |
| *Morganella morganii* IID 602 | 0.10 | 0.20 | 0.78 | 0.20 |
| *Serratia marcescens* IID 618 | 0.78 | 0.20 | 0.78 | 1.56 |
| *Haemophilus influenzae* ATCC 19418 | 0.20 | 0.05 | 0.39 | 0.39 |

(Note)

A: The compound obtained in Example 1(d)

B: Cefixime (sodium salt; known compound)

C: Cefnidir (sodium salt; known compound)

D: 7β-{2-(2-aminothiazol-4-yl)-2-[(Z)-hydroxyimino]acetamido}-3-allylthio-3-cephem-4-carboxylic acid sodium salt

Experiment 2

Three male ICR strain mice (4 weeks old) for each group were administered orally with the test drug, and the change of the concentration of the drug in blood was measured with the lapse of time. Dose of drug: 20 mg/kg (suspended in 5% gum arabic) Quantitative method: Bioassay method (test bacteria: Bacillus cereus S1101)

The results are shown in Table 2.

Table 2

| Test drug / Time after administration (hour) | E | F |
|---|---|---|
| 0.25 | 14.0 | 1.8 |
| 0.5 | 18.4 | 2.0 |
| 1.0 | 15.4 | 2.5 |
| 2.0 | 8.4 | 1.2 |
| 4.0 | 4.0 | 0.1 |

(Note)

E: The compound obtained in Example 2

F: Cefnidir (sodium salt)

Experiment 3

The mortality after a single dose of the test drug was measured using 3 male ICR strain mice (4 weeks old) for each group.

Administration route : intraperitoneal
Administration volume : 50 ml/kg (saline)
Dose : 4,000 mg/kg
Observation period : for 14 days after the administration
The results are shown in Table 3.

Table 3

| Test drug | G | H |
|---|---|---|
| Mortality | 0/3 | 2/3 |

(Note)
G: The compound obtained in Example 1(d)
H: 7β-{2-(2-aminothiazol-4-yl)-2-[(Z)-hydroxyimino]acetamido}-3-allylthio-3-cephem-4-carboxylic acid sodium salt

## Claims

**1.** A cephalosporin derivative represented by the formula:

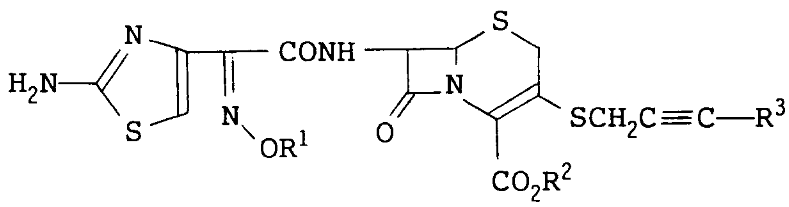

(wherein $R^1$ and $R^3$ are each a hydrogen atom or a lower alkyl group, and $R^2$ is a hydrogen atom or an in vivo hydrolyzable group) or non-toxic salts thereof.

**INTERNATIONAL SEARCH REPORT**

International application No.
PCT/JP93/00816

**A. CLASSIFICATION OF SUBJECT MATTER**

Int. $Cl^5$ C07D501/59, A61K31/545

According to International Patent Classification (IPC) or to both national classification and IPC

**B. FIELDS SEARCHED**

Minimum documentation searched (classification system followed by classification symbols)

Int. $Cl^5$ C07D501/59, A61K31/545

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| X | JP, A, 62-103093 (Taisho Pharmaceutical Co., Ltd.), May 13, 1987 (13. 05. 87), Claim; Tables 3, 4, 5 & EP, A, 210078 & US, A, 4812562 | 1 |
| Y | JP, A, 52-83492 (CIBA-Geigy AG.), July 12, 1977 (12. 07. 77), Claim, page 10 & US, A, 4256739 & DE, A, 2537974 & FR, A, 2282895 | 1 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| August 12, 1993 (12. 08. 93) | September 7, 1993 (07. 09. 93) |
| Name and mailing address of the ISA/ Japanese Patent Office | Authorized officer |
| Facsimile No. | Telephone No. |